# EUROPÄISCHE PATENTANMELDUNG

(11) **EP 1 454 894 A1**
(43) Veröffentlichungstag der Anmeldung: **08.09.2004**
(21) Anmeldenummer: 04000686.8
(22) Anmeldetag: 15.01.2004
(51) Int. Cl.: C07C 51/02, C07C 51/42, C07C 59/255

(54) **Verfahren zur kontinuierlichen Gewinnung freier Weinsäure aus Kaliumhydrogentartrat enhaltenden Rohstoffen**

(30) Priorität: 26.02.2003 DE 10308045
(71) Anmelder: Lurgi AG, 60295 Frankfurt am Main (DE)
(72) Erfinder: Stein, Dieter, 65191 Wiesbaden (DE); Bönsch, Rudolf, 55130 Mainz (DE); Erb, Klaus, 38640 Goslar (DE)
(74) Vertreter: Meyer-Dulheuer, Karl-Hermann, Dr.

(57) **Zusammenfassung**

Bei einem Verfahren zur kontinuierlichen Gewinnung freier Weinsäure aus Kaliumhydrogentartrat enthaltenden Rohstoffen werden diese mit Wasser gemischt und das Kaliumhydrogentartrat gelöst. Das Verfahren wird dadurch verbessert, dass die Suspension dekantiert, die geklärte Flüssigkeit nachgefiltert, das Filtrat unter Vakuum auf Kristallisationstemperatur abgekühlt, die gebildeten Kaliumhydrogentartrat-Kristalle gelöst, die Lösung einem Kationenaustausch unterworfen und die entstandene Weinsäure-Lösung eingedampft wird.

## Beschreibung

Die Erfindung betrifft ein Verfahren zur kontinuierlichen Gewinnung freier Weinsäure (H₆C₄O₆) aus wenigstens 5.0 Gew.-% Kaliumhydrogentartrat (KH₅C₄O₆) i.d.Tr. (in der Trockenmasse) enthaltenden Rohstoffen, insbesondere aus bei der Weinbereitung anfallenden Nebenprodukten, wie Weinstein, Weinhefe oder dergl., indem die Rohstoffe mit Wasser gemischt werden und Kaliumhydrogentartrat gelöst wird.

Der größte Teil der in der Industrie verwendeten Weinsäure wird aus bei der Weinbereitung anfallenden Kaliumhydrogentartrat enthaltenden Nebenprodukten, wie Weinstein, Weinhefe, Trester, Destillationsrückstände, Calciumtartrat etc. erzeugt. Bei den bekannteren Verfahren wird Kaliumhydrogentartrat durch Fällung gewonnen; entweder werden die zu verarbeitenden Rohstoffe mit einer Säure angegriffen und aus der erhaltenen Lösung Kaliumhydrogentartrat durch ein basisches Mittel ausgefällt, oder die Rohstoffe werden mit einem basischen Mittel behandelt und das Kaliumhydrogentartrat durch Zugabe von Säure ausgefällt.

Bei dem in der DE-C-264005 beschriebenen Verfahren werden die zu verarbeitenden Rohstoffe geröstet und mit basischen und sauren Mitteln in der Weise behandelt, dass eine Lösung eines neutralen Tartrats und eine Lösung von freier Weinsäure entsteht. Durch das Vermischen beider Lösungen wird die Weinsäure als Kaliumhydrogentartrat gefällt. Aus der FR-A-2646421 ist ein Verfahren zur Herstellung von Weinsäure bekannt, bei dem die in den Rohstoffen enthaltenen Bitartrate zu löslichen Tartraten neutralisiert werden. Die wässrigen Lösung der Tartrate wird zum Zwecke der Erzeugung einer Weinsäure-Lösung einer Elektrodialyse, bei der der Ionenaustausch an semipermeablen Membranen erfolgt, unterworfen. Bau und Betrieb einer solchen Elektrodialyse-Anlage bedürfen eines nicht unbeachtlichen Aufwands. Darüber hinaus müssen die dem Ionenaustausch unterworfenen Tartrat-Lösungen frei von organischen Substanzen sein, da sich diese sonst bei der Elektrodialyse unter Bildung von Ammoniak zersetzen.

Die DE-A-19819884 befasst sich mit einem Verfahren zur Erzeugung von Weinsäure aus Rohstoffen, deren Trockensubstanz zu mindestens 5.0 Gew.-% aus Kaliumhydrogentartrat besteht, indem zunächst durch Verrühren mit wässriger Kalilauge Kaliumhydrogentartrat zu Dikaliumtartrat (K₂H₄C₄O₆) umgesetzt wird, die gebildete wässrige Lösung nach Entfernung von Verunreinigungen durch Filtration in einer Fällungsstufe mit zudosierter Säure, vorzugsweise aus dem Prozess rückgeführter Weinsäure, bei einem pH-Wert von 2 bis 5 verrührt, aus der gebildeten Kaliumhydrogentartrat-Kristalle enthaltenden Suspension die Kaliumhydrogentartrat-Kristalle abgetrennt, mit Wasser gewaschen, eine zu wenigstens 80 Gew.-% mit Kaliumhydrogentratrat gesättigte wässrige Lösung erzeugt, aus dieser Lösung Kalium entfernt und aus der gebildeten wässrigen Weinsäure-Lösung Wasser wenigstens teilweise entfernt wird. Bei der Umsetzung von Kaliumhydrogentartrat mittels wässriger Kalilauge zu Dikaliumtartrat werden in nachteiliger Weise auch die darin enthaltenen organischen Verbindungen, wie Phenolate, Zuckerpolymere, Schleimstoffe und sonstige Zellinhaltsstoffe, gelöst, die die Filtration sehr stark behindern.

Es ist die Aufgabe der vorliegenden Erfindung, das vorstehend beschriebene Verfahren zur Gewinnung freier Weinsäure aus wenigstens 5.0 Gew.-% Kaliumhydrogentartrat i.d.Tr. enthaltenden Rohstoffen, insbesondere aus bei der Weinbereitung anfallenden Nebenprodukten, wie Weinstein, Weinhefe oder dergl., in der Weise zu gestalten, dass der bei der Filtration der wässrigen Lösung aus den abzutrennenden Verunreinigungen entstehende Filterkuchen die Bildung des Filtrats nicht behindert.

Diese Aufgabe wird dadurch gelöst, dass eine Suspension (Feststoff-Flüssigkeits-Gemisch), bestehend aus Feststoffen und in Wasser gelöstem Kaliumhydrogentartrat dekantiert, die dabei anfallende geklärte Flüssigkeit einer Mikrofiltration zur Abtrennung feinster Feststoff-Partikel zugeführt, das dabei gebildete Filtrat zum Zwecke der Kristallisation des Kaliumhydrogentartrats unter Vakuum auf Kristallisationstemperatur abgekühlt, die gebildeten Kaliumhydrogentartrat-Kristalle zum Zwecke des Abschleuderns von Flüssigkeit zentrifugiert und anschließend in Wasser gelöst, aus der wässrigen Kaliumhydrogetartrat-Lösung das Kalium durch Ionenaustausch entfernt und die entstandene Weinsäure-Lösung unter Bildung von Weinsäure-Kristallen eingedampft wird. Indem Kaliumhydrogentartrat mittels Wasser aus den Rohstoffen gelöst wird, werden die in den Rohstoffen enthaltenen organischen Verunreinigungen weitgehend nicht gelöst und bilden sowohl beim Dekantieren als auch bei der Mikrofiltration der beim Dekantieren entstandenen geklärten Flüssigkeit einen Filterkuchen, der die Abtrennung des Filtrats nicht beeinträchtigt. Durch die unter Vakuum durchgeführte Kühlkristallisation des Filtrats kann auf die Zugabe von Säure, insbesondere auf die Rückführung von Prozess-Weinsäure verzichtet werden. In vorteilhafter Weise ergibt sich dadurch die Möglichkeit, sowohl die Anlage für den Kationenaustausch als auch diejenige für das Eindampfen der Weinsäure-Lösung um 50 % kleiner als die entsprechenden Anlagen, die für die Durchführung des Verfahrens gemäß DE-A-19819884 erforderlich sind, auszulegen.

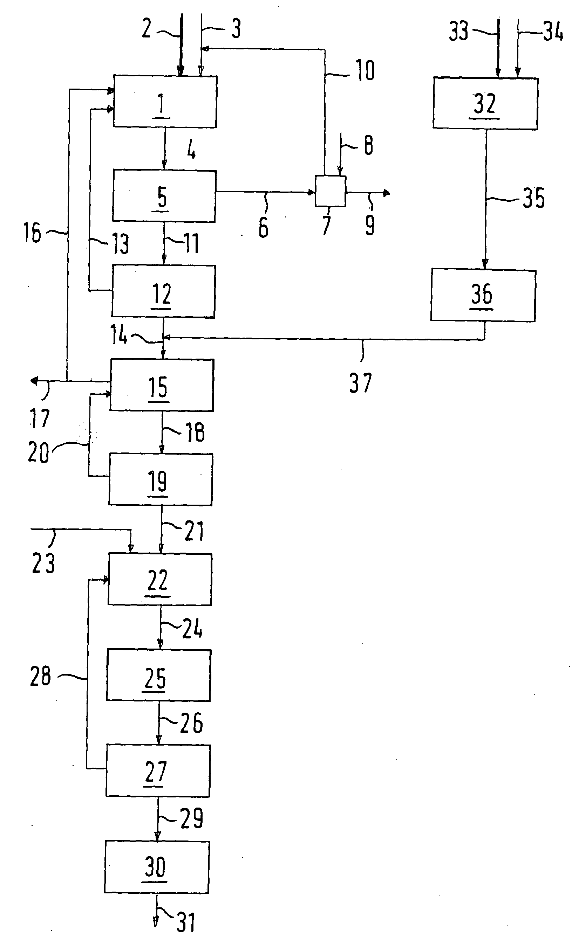

Die Erfindung ist an Hand des in der Zeichnung dargestellten Grundfließschemas näher und beispielhaft erläutert.

Einem beheizbaren Rührbehälter (1) wird über Leitung (2) als Kaliumhydrogentartrat enthaltender Rohstoff Weinhefe und über Leitung (3) Wasser aufgegeben und intensiv vermischt. Die dabei gebildet Suspension (Feststoff-Flüssigkeits-Gemisch) wird über Leitung (4) aus dem Rührbehälter (1) ausgetragen und einem Dekanter (5) zugeführt, durch den die überwiegend aus Weinhefe bestehenden Feststoffe von der Flüssigkeit getrennt werden. Die über Leitung (6) aus dem Dekanter (5) ausgetragenen Feststoffe werden in einem als Wascheinrichtung dienenden weiteren Dekanter (7), dem über Leitung (8) Wasser zugleitet wird, gereinigt, über Leitung (9) abgezogen und einer weiteren Verwendung, z.B. als Futterhefe, zugeführt. Das anfallende Waschwasser wird über Leitung (10) in die Leitung (3) zurückgeführt. Die aus dem Dekanter (5) über Leitung (11) frei ablaufende geklärte Flüssigkeit wird einer keramischen Mikrofiltereinrichtung (12) zugeleitet, um in der geklärten Flüssigkeit eventuell noch vorhandene Feststoffteilchen, die über Leitung (13) in den Rührbehälter (1) zurückgeführt werden, abzutrennen. Das gelöste Kaliumhydrogentartrat enthaltende Filtrat wird über Leitung (14) einem aus einer oder mehreren Stufen bestehenden Kristallisator (15) zugeführt, in dem durch Abkühlung des Filtrats auf eine Temperatur von 5 °C bei einem Vakuum von 0.007 bar Kaliumhydrogentartrat auskristallisiert. Falls erforderlich, besteht die Möglichkeit zur Entfärbung des Filtrats zwischen der keramischen Mikrofiltereinrichtung (12) und Kristallisator (15) einen nicht dargestellten Aktivkohlefilter anzuordnen. Die bei der Kühlkristallisation gebildete Flüssigkeit wird teilweise über Leitung (16) in den Rührbehälter (1) zurückgeleitet, während der verbleibende Anteil aus dem Prozess über Leitung (17) ausgeschleust wird. Die über Leitung (18) aus dem Kristallisator (15) abgeführten Kaliumhydrogentartrat-Kristalle werden einer Zentrifuge (19), in der die den Kaliumhydrogentartrat-Kristallen noch anhaftende Flüssigkeit abgeschleudert wird, zugeführt. Die dabei anfallende Flüssigkeit wird über Leitung (20) in den Kristallisator (15) zurückgeführt. Die über Leitung (21) die Zentrifuge (19) verlassenden Kaliumhydrogentartrat-Kristalle werden einem beheizbaren Rührbehälter (22) aufgegeben, in dem die Kaliumhydrogentartrat-Kristalle bei Temperaturen von 75 - 95 °C unter Zusatz von über Leitung (23) zuströmendem Wasser aufgelöst werden. Aus dem Rührbehälter (22) wir die weitgehend mit Kaliumhydrogentartrat gesättigte Lösung über Leitung (24) abgezogen, dann zum Entfärben über ein Aktivkohlebett (25) geleitet und danach über Leitung (26) einem Kationen-Austauscher (27) zur Entfernung der K⁺-lonen zugeführt. Ein Anteil der 10 bis 20 % Weinsäure enthaltenden Lösung wird aus dem Kationen-Austauscher (27) über Leitung (28) in den Rührbehälter (22) zurückgeleitet, während der restliche Anteil der Weinsäure-Lösung über Leitung (29) in einen Eindampfer (30) eintritt und in diesem unter Bildung von Weinsäure-Kristallen eingedampft wird. Die gebildeten Weinsäure-Kristalle werden über Leitung (31) aus dem Prozess ausgeschleust.

Eine vorteilhafte Ausgestaltung dieses Grundfließschematas besteht darin, dass in einem beheizbaren Rührbehälter (32) über Leitung (33) zugeführter Weinstein und über Leitung (34) zulaufendes Wasser gemischt werden, das Gemisch über Leitung (35) einer keramischen Mikrofiltereinrichtung (36) aufgegeben und das gebildete Filtrat über Leitung (37) in die zum Kristallisator (15) führende Leitung (14) eingespeist wird.

Bei der Weinbereitung angefallene einen Kaliumhydrogentartrat-Gehalt i.d.Tr. von 8.5 Gew.-% aufweisende Weinhefe wird in dem Rührbehälter (1) mit 3.0 kg Wasser pro kg Weinhefe intensiv bei einer Temperatur von ca. 80 °C max. 60 min lang gemischt, um das Kaliumhydrogentartrat vollständig aus der Weinhefe zu lösen. Anschließend wird die eine Temperatur von ca. 80 °C besitzende wässrige Lösung mittels eines Dekanters (5) in einen aus festen organischen Partikeln bestehenden Bodenkörper mit 30 Gew.-% i.d.Tr. und eine 2.8 Gew-% Kaliumhydrogentartrat enthaltende geklärte Flüssigkeit getrennt. Nach dem Durchlauf dieser Flüssigkeit durch einen Keramik-Filter (12) wird aus dem Filtrat in einem Kristallisator (15) bei einer Temperatur von ca. 5 °C und einem Vakuum von 0.007 bar Kaliumhydrogentartrat auskristallisiert. Die mittels einer Zentrifuge (19) von Flüssigkeit weitgehend befreiten Kaliumhydrogentartrat-Kristalle werden unter Zusatz von Wasser in einem Rührbehälter (22) gelöst und die Lösung einem Kationen-Austauscher (27) aufgegeben, in dem 15%ige Weinsäure erzeugt, die anschließend in einem Verdampfer (30) unter Bildung von Weinsäure-Kristallen eingedampft wird.

## Patentansprüche

1. Verfahren zur kontinuierlichen Gewinnung freier Weinsäure aus wenigstens 5.0 Gew.-% Kaliumhydrogentartrat i.d.Tr. (in der Trockenmasse) enthaltenden Rohstoffen, insbesondere aus bei der Weinbereitung anfallenden Nebenprodukten, wie Weinhefe, Weinstein oder dergl., indem die Rohstoffe mit Wasser gemischt werden und Kaliumhydogentartrat gelöst wird, **dadurch gekennzeichnet, dass** die Suspension (Feststoff-Flüssigkeits-Gemisch) dekantiert, die geklärte Flüssigkeit einer Mikrofiltration unterworfen, das Filtrat unter Vakuum auf Kristallisationstemperatur abgekühlt, die gebildeten Kaliumhydrogentartrat-Kristalle zentrifugiert und in Wasser gelöst, aus der wässrigen Kaliumhydrogentartrat-Lösung das Kalium durch Ionenaustausch entfernt und die entstandene Weinsäure-Lösung unter Bildung von Weinsäure-Kristallen eingedampft wird.

2. Verfahren nach Anspruch 1, **dadurch gekennzeichnet, dass** das durch eine Mikrofiltration wässriger Weinstein-Lösung gewonnene Filtrat dem für die Kühlkristallisation vorgesehnen Filtrat zugesetzt wird.

3. Verfahren nach einem der Ansprüche 1 und 2, **dadurch gekennzeichnet, dass** die beim Dekantieren und/oder bei der Mikrofiltration und/oder bei Kühlkristallisation und/oder beim Zentrifugieren anfallende Flüssigkeit, wenigstens teilweise in den Prozess, vorzugsweise in die aus Feststoffen und in Wasser gelöstem Kaliumhydrogentartrat bestehende Suspension, zurückgeführt wird.

4. Verfahren nach einem der Ansprüche 1 bis 3, **dadurch gekennzeichnet, dass** die Kühlkristallisation bei einer Temperatur von 15 bis 5 °C und einem Vakuum von 0.015 bis 0.007 bar durchgeführt wird.

5. Vorrichtung zur kontinuierlichen Gewinnung freier Weinsäure aus wenigstens 5.0 Gew.-% Kaliumhydrgentartrat i.d.Tr. enthaltenden Rohstoffen, insbesondere aus bei der Weinherstellung anfallenden Nebenprodukten, wie Weinhefe, Weinstein oder dergl., indem die Rohstoffe mit Wasser gemischt werden und Kaliumhydrogentartrat gelöst wird, die Suspension dekantiert, die geklärte Flüssigkeit einer Mikrofiltration unterworfen, das Filtrtat unter Vakuum auf Kristallisationstemperatur abgekühlt, die gebildeten Kaliumhydrogentartrat-Kristalle zentrifugiert und in Wasser gelöst, aus der wässrigen Kaliumhydrogentartrat-Lösung das Kalium durch Ionenaustausch entfernt und die entstandene Weinsäure-Lösung unter Bildung von Weinsäure-Kristallen eingedampft wird, **gekennzeichnet durch** einen die Suspension enthaltenden beheizbaren Behälter (1) mit Rührwerk, einen Dekanter (5) mit Schnecken- oder Siebaustrag, eine keramische Mikrofiltereinrichtung (12) mit einer Porenweite von 0.05 bis 0.6 µm, einen Kühlungskristallisator (15) zum Kristallisieren der Kaliumhydrogentartrat-Kristalle, eine Zentrifuge (19) zum Abschleudern der Flüssigkeit von den Kaliumhydrogentartrat-Kristallen, einen beheizbaren Behälter (22) zum Auflösen der Kaliumhydrogentartrat-Kristalle, einen Kationenaustauscher (27) und einen Eindampfer (30) zum Entfernen des Wassers aus der Weinsäure-Lösung.
